# FASCICULE DE BREVET EUROPEEN

(11) **EP 1 799 628 B1**
(45) Date de publication et mention de la délivrance du brevet: **24.06.2015**
(21) Numéro de dépôt: 05804710.1
(22) Date de dépôt: 06.10.2005
(51) Int. Cl.: C07C 27/12, C07C 29/50, C07B 41/02, C07B 41/06, C07B 41/14, C07C 49/403, C07C 45/33

(54) **PROCÉDÉ D'OXYDATION PAR L'OXYGÈNE D'HYDROCARBURES CYCLIQUES SATURÉS**
VERFAHREN ZUR OXIDATION VON GESÄTTIGTEN ZYKLISCHEN KOHLENWASSERSTOFFEN MIT SAUERSTOFF
METHOD FOR OXIDATION OF SATURATED CYCLIC HYDROCARBONS WITH OXYGEN

(30) Priorité: 12.10.2004 FR 0410731
(43) Date de publication de la demande: 27.06.2007
(73) Titulaire: Rhodia Chimie SA, 92512 Boulogne-Billancourt Cedex (FR)
(72) Inventeur: VERACINI, Serge, F-69005 LYON (FR); AUGIER, Frédéric, F-69360 SAINT SYMPHORIEN D'OZON (FR)
(74) Mandataire: Chatelan, Florence Anne
(86) Numéro de dépôt international: PCT/FR2005/002461
(87) Numéro de publication internationale: WO 2006/040442

(56) Documents cités:
- WO-A-03/031051
- US-A- 6 008 415

## Description

La présente invention concerne un procédé en continu d'oxydation par l'oxygène d'hydrocarbures cycliques saturés pour obtenir un mélange d'hydroperoxydes, d'alcools et de cétones.

Elle concerne plus particulièrement un procédé d'oxydation de cyclohexane mis en oeuvre dans une colonne formant un réacteur à bulles, pour la formation d'hydroperoxyde de cyclohexyle, de cyclohexanol et cyclohexanone.

Cette étape d'oxydation forme le première étape d'un procédé de fabrication d'acide adipique, par exemple.

Dans les procédés de fabrication d'acide adipique, un des plus utilisés consiste à oxyder par l'oxygène moléculaire, le cyclohexane en hydroperoxyde de cyclohexyle puis à décomposer catalytiquement cet hydroperoxyde en un mélange de cyclohexanone et cyclohexanol. Ce mélange est ensuite oxydé en acide adipique par une oxydation nitrique.

Cette première étape d'oxydation du cyclohexane est généralement réalisée en milieu biphasique liquide/gaz, le gaz oxydant étant introduit sous forme de bulles dans le milieu liquide, dans des réacteurs tubulaires appelés réacteurs à bulles ou dans des réacteurs agités tels que décrit dans le brevet US 6 008 415. Dans ce brevet, la sécurité du procédé, notamment le contrôle de la concentration en oxygène dans le ciel du réacteur est réalisé par injection d'un gaz inerte dans le ciel du réacteur ou la partie de liquide dégazéifiée.

Plusieurs procédés ont déjà été proposés dans lesquels les flux de gaz oxydant et flux de liquide peuvent être à co-courant ou à contre courant dans le réacteur.

Le procédé de l'invention concerne les modes de réalisation avec des flux liquide et gazeux à co-courants dans le réacteur tubulaire.

Dans ce type de procédé, la phase liquide est dégazéifiée en haut de colonne pour former un ciel gazeux et récupérer une phase liquide exempte de gaz ou sensiblement exempte de gaz. Ce ciel gazeux est composé du gaz alimenté n'ayant pas réagi et notamment de l'oxygène non consommé ainsi que des vapeurs de l'hydrocarbure et autres produits organiques. La concentration en hydrocarbure et autres produits organiques est déterminée par la tension de vapeur de ces composés aux conditions de température et de pression utilisées.

Pour prévenir une explosion de ce mélange de gaz et vapeur, il est nécessaire que la concentration volumique en oxygène par rapport au volume de gaz dans l'enceinte à l'exception de l'hydrocarbure soit inférieure à une certaine limite. Ainsi, dans le cas d'un mélange oxygène, azote, cyclohexane cette limite supérieure est de 8,5% d'oxygène par rapport au volume d'oxygène et d'azote. Ainsi, dans ce domaine de concentration en oxygène, le mélange gazeux est toujours dans un domaine de non explosivité quelle que soit la concentration en vapeur d'hydrocarbure, par exemple de cyclohexane, et autres composés organiques. Ces limites de concentration en oxygène sont soit publiées et connues de l'homme du métier pour certains systèmes déjà exploités comme le système oxygène / azote / cyclohexane soit facilement déterminable par l'homme du métier par application des méthodes connues et publiques de détermination des limites d'explosivité de mélange gazeux. Ainsi, pour chaque système particulier, l'homme du métier déterminera cette limite supérieure de concentration en oxygène par les techniques habituelles avant de réaliser l'oxydation de l'hydrocarbure. Pour plus de clarté, on désignera dans le présent texte cette limite de concentration comme la concentration limite supérieure en oxygène.

Actuellement, cette règle de sécurité est respectée en, par exemple, contrôlant la quantité d'oxygène alimentée dans le réacteur.

De ce fait, il est impossible actuellement d'alimenter le réacteur avec une quantité d'oxygène élevée et d'autre part da concentration en oxygène diminue au cours du trajet de la phase gaz dans le réacteur tubulaire.

Cette spécification sur la quantité d'oxygène alimentée dans le réacteur, et notamment sa concentration dans la phase gaz ne permet pas d'obtenir une cinétique élevée de la réaction d'oxydation. Cette concentration faible en oxygène affecte également la sélectivité en hydroperoxyde de la réaction d'oxydation.

En outre, pour effectuer un contrôle efficace de la concentration volumique en oxygène dans le ciel du réacteur, il est connu d'alimenter la totalité de l'oxygène en bas de colonne. De ce fait, la concentration ou pression partielle en oxygène diminue sur la longueur du réacteur, ne permettant pas d'avoir une cinétique de réaction élevée dans tout le réacteur.

Un des buts de l'invention est de remédier à ces inconvénients en proposant un procédé permettant de s'assurer que la concentration volumique en oxygène dans le ciel du réacteur sera inférieure à la concentration 8,5 % quelle que soit la concentration ou pression partielle en oxygène présente dans la phase liquide contenue dans le réacteur.

A cet effet, l'invention propose un procédé continu d'oxydation par l'oxygène, d'un hydrocarbure cyclique saturé en un mélange d'hydroperoxyde, d'alcool et de cétone dans un réacteur tubulaire à bulles, consistant à alimenter dans ledit réacteur, en bas de colonne, un flux liquide d'hydrocarbure à oxyder et un flux gazeux contenant de l'oxygène, ledit flux gazeux étant introduit sous forme de bulles de gaz, à faire circuler dans ladite colonne le flux liquide contenant les bulles de gaz, en haut de colonne à dégazéifier la phase liquide avec formation d'un ciel de gaz dans la partie haute de la colonne, et à soutirer la phase liquide contenant les produits de réaction au niveau de la zone de dégazéification.

Le procédé de l'invention se caractérise en ce qu'il consiste à alimenter un gaz contenant de l'oxygène à différents étages de la colonne et à alimenter dans le réacteur un gaz non oxydant, dans la phase liquide au niveau de la zone de dégazéification ou immédiatement en amont, et/ou dans le ciel gazeux du réacteur, selon un débit suffisant pour maintenir une concentration volumique en oxygène dans le ciel du réacteur à une valeur inférieure ou égale à la concentration limite supérieure en oxygène. Dans le cas où l'hydrocarbure est le cyclohexane et le gaz oxydant est un mélange azote oxygène, cette limite est de 8,5%. Avantageusement le débit de gaz non oxydant est déterminé pour obtenir dans le ciel du réacteur une concentration en oxygène inférieure à environ 30% de la concentration limite supérieure en oxygène. Ainsi, dans le cas où l'hydrocarbure est le cyclohexane, le débit de gaz oxydant est déterminé pour obtenir une concentration en oxygène dans le ciel du réacteur à une valeur inférieure ou égale à 5%.

Le gaz non oxydant est avantageusement choisi parmi l'azote, les gaz inertes, l'air appauvri en oxygène.

Selon une autre caractéristique de l'invention, les hydrocarbures saturés cycliques sont choisis parmi le cyclohexane, la décaline et le cyclododécane.

Selon l'invention, l'alimentation d'une quantité déterminée de gaz non oxydant dans le ciel gazeux du réacteur permet de contrôler de manière certaine que la concentration volumique en oxygène dans ce ciel sera toujours inférieure à une certaine valeur, à savoir 8,5% dans le cas ou l'hydrocarbure à oxyder est le cyclohexane et les gaz sont l'oxygène et l'azote.

Cette quantité de gaz non oxydant alimenté dans le ciel est déterminée en fonction de la quantité d'oxygène alimentée dans le réacteur tubulaire.

Ainsi, on peut déterminer la quantité maximale de gaz non oxydant à injecter pour obtenir une concentration en oxygène inférieure à 8,5%, dans le cas où tout l'oxygène injecté dans la colonne se retrouve dans le ciel du réacteur, c'est-à-dire que la réaction d'oxydation ne se soit pas produite. Cette quantité est bien entendu la quantité maximale de gaz inerte que l'on peut introduire. Des quantités plus faibles peuvent être alimentées en prenant en compte la consommation de l'oxygène dans la colonne.

Le procédé de l'invention permet également d'alimenter une quantité supérieure d'oxygène dans la colonne, notamment en alimentant un gaz à teneur en oxygène élevée tel que par exemple, un air enrichi en oxygène ou même de l'oxygène pur. Comme la pression partielle en oxygène sera plus élevée dans les bulles de gaz dispersées dans le liquide, la cinétique de la réaction d'oxydation est augmentée. Cette cinétique plus élevée s'accompagne d'une sélectivité de l'oxydation en hydroperoxyde de cyclohexyle plus élevée.

Le procédé de l'invention permet également d'alimenter l'oxygène ou le gaz contenant de l'oxygène en différents points sur la longueur de la colonne ainsi maintenue à une pression partielle en oxygène dans les bulles de gaz la plus élevée possible sur sensiblement toute la zone de réaction de la colonne. En effet, il n'est pas nécessaire que la concentration en oxygène dans les bulles arrivant dans le ciel du réacteur soit très faible, car l'oxygène arrivant dans le ciel gazeux sera dilué dans le gaz non oxydant alimenté conformément à l'invention.

De ce fait, il est possible avec le procédé de l'invention d'obtenir une cinétique de la réaction d'oxydation élevée dans toute la zone réactive de la colonne.

Selon un mode de réalisation particulier de l'invention, le réacteur tubulaire comprend des plateaux divisant le réacteur en plusieurs étages. Ces plateaux sont perforés pour permettre la circulation du liquide et des bulles de gaz sans accumulation ou formation de ciel gazeux au niveau de chaque plateau. De tels réacteurs sont déjà connus et un mode de réalisation d'un réacteur comprenant de plateaux perforés est décrit dans la demande de brevet WO03/031051

Le gaz contenant l'oxygène peut être alimenté en totalité en bas de colonne ou alimenté en plusieurs points de la colonne, avantageusement au niveau de chaque étage défini par les plateaux.

Dans le mode de réalisation consistant à alimenter le gaz contenant l'oxygène en plusieurs points de la colonne, la concentration en oxygène dans le gaz alimenté peut être identique ou différente pour chaque point d'alimentation. De même, les quantités de gaz et d'oxygène peuvent également être identiques ou différentes à chaque point d'alimentation. Avantageusement, la teneur en oxygène dans le gaz oxydant alimenté en bas de colonne est élevée et va en décroissant depuis le bas de la colonne vers le haut de la colonne pour les autres points d'alimentation du gaz oxydant.

Selon un mode de réalisation de l'invention, le gaz non oxydant est avantageusement alimenté dans la phase liquide, en amont immédiat du dégazeur. En effet, l'alimentation de ce gaz favorise le mélange entre les bulles de gaz contenant de l'oxygène et le gaz inerte. Ainsi l'homogénéité de la teneur en oxygène est assurée avant l'arrivée du gaz dans le ciel gazeux.

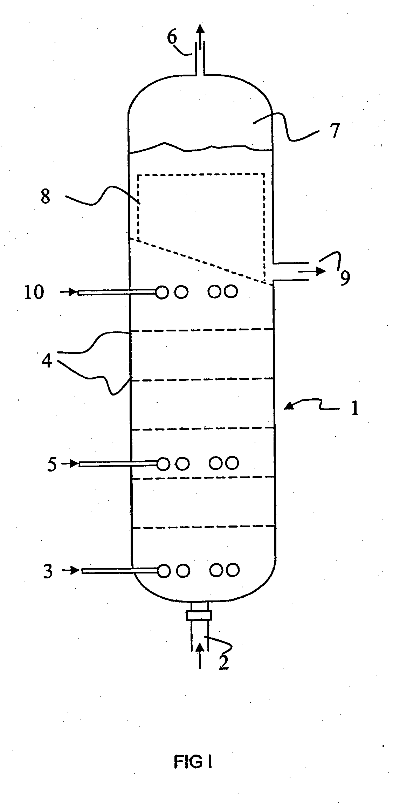

D'autres détails, avantages de l'invention apparaîtront plus clairement au vu des exemples donnés ci-dessous uniquement à titre indicatif et dont la description est faite en référence à la figure annexée représentant schématiquement un mode de réalisation de principe d'un réacteur à bulles selon le procédé de l'invention.

Le procédé de l'invention est mis en oeuvre dans un réacteur (1) tubulaire à bulles comprenant une alimentation en hydrocarbure à oxyder (2) disposée en fond de colonne.

Le réacteur comprend également une alimentation en gaz oxydant (3) disposée également en pied de colonne. Cette alimentation en gaz oxydant comprend un dispositif non illustré permettant d'alimenter le gaz sous forme de bulles qui se dispersent dans la phase liquide.

Dans le mode de réalisation illustré, le réacteur (1) ou colonne comprend des plateaux ou parois horizontales perforées (4) divisant la colonne en plusieurs étages.

Le réacteur (1) illustré comprend également d'autres alimentations en gaz oxydant (5) disposées au niveau de certains étages définis par les plateaux (4). Ces alimentations (5) sont avantageusement identiques à l'alimentation (3).

En tête de colonne, le réacteur (1) comprend une sortie des gaz (6) permettant au gaz de ciel formé en tête de colonne (7) d'être évacué.

Selon le mode de réalisation illustré, le réacteur est équipé d'un dégazeur (8) formé par une cuve immergée dans la phase liquide juste au dessous du niveau supérieur de la phase liquide.

La phase liquide pénètre dans cette cuve par déversement. La cuve comprend une évacuation (9) du liquide vers l'extérieur de la colonne. Le liquide ainsi recueilli par le tube d'évacuation (9) comprend les composés oxydés sans bulles de gaz dispersées.

Selon l'invention, le réacteur est équipé d'une alimentation (10) débouchant dans le mode de réalisation illustré, au niveau du dernier étage en amont du dégazéifieur

Par cette alimentation, du gaz non oxydant est alimenté pour ainsi maintenir et contrôler la concentration en oxygène dans le ciel (7) du réacteur.

D'autres détails, avantages de l'invention apparaîtront plus clairement au vu des exemples donnés uniquement à titre d'illustration et sans effet de limitation de l'invention et en référence à la figure unique annexée qui représente un schéma synoptique d'un mode de réalisation d'un réacteur utilisé pour la mise en oeuvre du procédé de l'invention.

### Exemples

Un essai d'oxydation de cyclohexane en un mélange d'hydroxyperoxyde de cyclohexyle, (HPOCH), cyclohexanone et cyclohexanol a été réalisé dans un réacteur (1) illustré à la figure unique.

Ce réacteur a un diamètre de 0,1 m, une hauteur de 8 m et comprend 5 plateaux (4) perforés.

La température dans le réacteur est de 184 °C et la pression absolue est de 22, 6 bars.

La colonne ou réacteur (1) comprend une alimentation de gaz oxydant (3) disposée en pied de colonne et une seconde alimentation de gaz inerte (10) disposée à environ 10 cm au-dessous de l'interface supérieur gaz/liquide, ou du niveau de liquide dans la colonne.

En (2), un flux de cyclohexane comprenant 0,2 % en poids d'hydroperoxyde de cyclohexyle est alimenté.

Le taux de transformation de cyclohexane dans le réacteur est de 4,5 %. Pour obtenir ce taux de transformation, on ajuste le débit d'alimentation en cyclohexane dans le réacteur. Le débit de gaz inerte ou non oxydant alimenté en (10) est déterminé pour obtenir dans le ciel gazeux (7) du réacteur un rapport volumique de O₂ par rapport au volume total N₂ + O₂ égal ou inférieur à 2%.

Les conditions et résultats obtenus pour différents essais sont listés dans le tableau I ci-dessous:

| Essai | Débit de cyclohexane (kg/h) | Nature et débit de gaz oxydant (kg/h) | Nature et débit de gaz non oxydant (kg/h) | Productivité kg/m³/h | Sélectivité en (HPOCH, cyclohexanone cyclohexanol) % |
|---|---|---|---|---|---|
| Comparatif | 293 | Air contenant | ○ | 136 | 86,5 |
| | | 21% O₂ | | | |
| | | 19 Kg/h | | | |
| 1 | 430 | Air contenant | Azote | 220 | 93,1 |
| | | 21% O₂ | 26 | | |
| | | 25 Kg/h | | | |
| 2 | 389 | Air contenant | Azote | 187 | 95,2 |
| | | 21% O₂ | 150 | | |
| | | 35 Kg/h | | | |
| 3 | 554 | Air contenant | Azote | 305 | 93,8 |
| | | 40% O₂ | 61 | | |
| | | 19 Kg/h | | | |

La productivité représente la quantité de produits oxydés récupérés par unité de temps et ramené à un volume de réacteur de 1 m³

Ces essais montrent que le procédé de l'invention permet d'augmenter la sélectivité en produits valorisables, c'est-à-dire transformables en acide adipique par exemple. Par sélectivité", on entend le rendement en produits valorisables divisé par le taux de transformation du produit à valoriser.

Ils démontrent également l'augmentation importante de productivité d'un réacteur donné. Ces résultats sont obtenus avec un respect strict des règles de sécurité.

En effet, le procédé de l'invention permet de transformer une quantité plus importante de cyclohexane dans un réacteur de même dimension. En effet, les débits de cyclohexane alimentés dans les essais 1 à 3 sont nettement supérieurs à celui de l'essai comparatif. Donc, la productivité de la réaction est augmentée, avec une amélioration de la sélectivité.

## Revendications

1. Procédé continu d'oxydation d'un hydrocarbure cyclique saturé en un mélange d'hydroperoxyde, alcool,cétone, par de l'oxygène consistant à introduire en fond d'une colonne et à co-courant un flux liquide d'hydrocarbure à oxyder et un flux gazeux contenant de l'oxygène, dégazer la phase liquide en tête de colonne par formation d'un ciel gazeux et à soutirer la phase liquide dégazifiée, **caractérisé en ce qu'**il consiste à alimenter un gaz contenant de l'oxygène à différents étages de la colonne et à alimenter dans le ciel et/ou dans la phase liquide au niveau de la zone de dégazéification ou immédiatement en amont, un flux de gaz non oxydant selon un débit suffisant pour maintenir la concentration en oxygène dans le ciel gazeux à une concentration volumique inférieure ou égale à la concentration limite supérieure en oxygène.

2. Procédé selon la revendication 1, **caractérisé en ce que** le gaz non oxydant est de l'azote, un gaz inerte ou de l'air appauvri en oxygène.

3. Procédé selon la revendication 1 ou 2, **caractérisé en ce que** l'hydrocarbure saturé est choisi dans le groupe comprenant le cyclohexane, la décaline, le cyclododécane.

4. Procédé selon la revendication 3, **caractérisé en ce que** la concentration volumique limite supérieure en oxygène est égale à 8,5 % quand l'hydrocarbure est le cyclohexane.

5. Procédé selon l'une des revendications précédentes, **caractérisé en ce que** la colonne comprend des plateaux perforés.

6. Procédé selon la revendication 5, **caractérisé en ce que** les quantités d'oxygène alimentées à chaque étage de la colonne sont égales.

7. Procédé selon la revendication 6, **caractérisé en ce que** les quantités d'oxygène alimentées à chaque étage de la colonne sont décroissantes par rapport au sens de circulation de la phase liquide dans la colonne.

8. Procédé selon l'une des revendications 6 à 7, **caractérisé en ce que** le gaz oxydant alimenté à différents étages contient une concentration en oxygène variable.

9. Procédé selon l'une des revendications précédentes **caractérisé en ce que** la gaz contenant de l'oxygène est choisi dans le groupe comprenant l'oxygène, l'air enrichi ou appauvri en oxygène.

## Patentansprüche

1. Kontinuierliches Verfahren zur Oxidation eines gesättigten cyclischen Kohlenwasserstoffs mit Sauerstoff zu einem Gemisch von Hydroperoxid, Alkohol und Keton, bei dem man am Sumpf einer Kolonne und im Gleichstrom einen zu oxidierenden flüssigen Kohlenwasserstoffstrom und einen Sauerstoff enthaltenden Gasstrom einträgt, die flüssige Phase am Kopf der Kolonne durch Bildung eines Kopfgasraums entgast und die entgaste flüssige Phase abzieht, **dadurch gekennzeichnet, dass** man verschiedenen Stufen der Kolonne ein Sauerstoff enthaltendes Gas zuführt und dem Kopfraum und/oder der flüssigen Phase auf Höhe der Entgasungszone oder unmittelbar stromaufwärts davon einen Strom von nicht oxidierendem Gas in einer Strömungsrate zuführt, die dazu ausreicht, die Sauerstoffkonzentration im Kopfgasraum bei einer Volumenkonzentration kleiner gleich der oberen Sauerstoffgrenzkonzentration zu halten.

2. Verfahren nach Anspruch 1, **dadurch gekennzeichnet, dass** es sich bei dem nicht oxidierenden Gas um Stickstoff, ein Inertgas oder sauerstoff-abgereicherte Luft handelt.

3. Verfahren nach Anspruch 1 oder 2, **dadurch gekennzeichnet, dass** der gesättigte Kohlenwasser-stoff aus der Gruppe umfassend Cyclohexan, Decalin und Cyclododecan ausgewählt wird.

4. Verfahren nach Anspruch 3, **dadurch gekennzeichnet, dass** die obere Sauerstoffgrenzkonzentration gleich 8,5% ist, wenn es sich bei dem Kohlenwasserstoff um Cyclohexan handelt.

5. Verfahren nach einem der vorhergehenden Ansprüche, **dadurch gekennzeichnet, dass** die Kolonne perforierte Böden umfasst.

6. Verfahren nach Anspruch 5, **dadurch gekennzeichnet, dass** die jeder Stufe der Kolonne zugeführten Sauerstoffmengen gleich sind.

7. Verfahren nach Anspruch 6, **dadurch gekennzeichnet, dass** die jeder Stufe der Kolonne zugeführten Sauerstoffmengen in der Strömungsrichtung der flüssigen Phase durch die Kolonne abnehmen.

8. Verfahren nach einem der Ansprüche 6 bis 7, **dadurch gekennzeichnet, dass** das in verschiedenen Stufen zugeführte oxidierende Gas eine variable Sauerstoffkonzentration enthält.

9. Verfahren nach einem der vorhergehenden Ansprüche, **dadurch gekennzeichnet, dass** das Sauerstoff enthaltende Gas aus der Gruppe umfassend Sauerstoff, sauerstoffangereicherte Luft und sauerstoffabgereicherte Luft ausgewählt wird.

## Claims

1. Continuous method for oxidizing a saturated cyclic hydrocarbon by oxygen to a mixture of hydroperoxide, alcohol and ketone, whereby a liquid stream of hydrocarbon to be oxidized and a gas stream containing oxygen are introduced in cocurrent flow at the bottom of a column, the liquid phase at the top of the column is degasified by forming an overhead and the degasified liquid phase is withdrawn, **characterized in that** a gas containing oxygen is supplied to various stages of the column and a stream of non-oxidizing gas is supplied to the overhead and/or in the liquid phase in the degasification zone or immediately upstream thereof, at a sufficient flow rate to maintain the oxygen concentration of the overhead at a volumetric concentration not exceeding the upper oxygen concentration limit.

2. Method according to Claim 1, **characterized in that** the non-oxidizing gas is nitrogen, an inert gas, or oxygen-depleted air.

3. Method according to either of Claims 1 and 2, **characterized in that** the saturated hydrocarbon is selected from the group comprising cyclohexane, decaline, and cyclododecane.

4. Method according to Claim 3, **characterized in that** the upper oxygen volumetric concentration limit is 8.5% if the hydrocarbon is cyclohexane.

5. Method according to one of the preceding claims, **characterized in that** the column comprises perforated trays.

6. Method according to Claim 5, **characterized in that** the quantities of oxygen supplied to each stage of the column are equal.

7. Method according to Claim 6, **characterized in that** the quantities of oxygen supplied to each stage of the column decrease along the flow direction of the liquid phase in the column.

8. Method according to either of Claims 6 and 7, **characterized in that** the oxidizing gas supplied at various stages contains a variable oxygen concentration.

9. Method according to one of the preceding claims, **characterized in that** the gas containing oxygen is selected from the group comprising oxygen, oxygen-enriched air and oxygen-depleted air.
